(19) Europäisches Patentamt
European Patent Office
Office européen des brevets

(11) Veröffentlichungsnummer: **0 301 339**
**A1**

# (12) EUROPÄISCHE PATENTANMELDUNG

(21) Anmeldenummer: **88111483.9**

(22) Anmeldetag: **16.07.88**

(51) Int. Cl.⁴: **C07D 231/44 , C07D 401/12 , A01N 43/56 , //(C07D401/12, 231:44,213:61)**

(30) Priorität: **28.07.87 DE 3724920**

(43) Veröffentlichungstag der Anmeldung:
**01.02.89 Patentblatt 89/05**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gehring, Reinhold, Dr.**
**Dasnoeckel 49**
**D-5600 Wuppertal 11(DE)**
Erfinder: **Jensen-Korte, Uta, Dr.**
**Geibelstrasse 9**
**D-4000 Düsseldorf 1(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal 1(DE)**
Erfinder: **Becker, Benedikt, Dr.**
**Metzkausener Strasse 14**
**D-4020 Mettmann(DE)**
Erfinder: **Homeyer, Bernhard, Dr.**
**Obere Strasse 28**
**D-5090 Leverkusen 3(DE)**

## (54) Substituierte 1-Aryl-5-(het)arylmethylamino-pyrazole.

(57) Es werden neue substituierte 1-Aryl-5-(het)arylmethylamino-pyrazole der allgemeinen Formel (I)

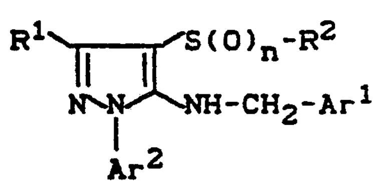

(I)

bereitgestellt,
in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$Ar^1$ für substituiertes Aryl oder für gegebenenfalls substituiertes Heteroaryl steht,
$Ar^2$ für jeweils gegebenenfalls substituiertes Phenyl oder Pyridyl steht und

EP 0 301 339 A1

n für eine Zahl 0, 1 oder 2 steht.
Die neuen Verbindungen der Formel (I) besitzen eine stark ausgeprägte insektizide Wirksamkeit.

## Substituierte 1-Aryl-5-(het)arylmethylamino-pyrazole

Die Erfindung betrifft neue substituierte 1-Aryl-5-(het)arylmethylamino-pyrazole, ein Verfahren zu ihrer Herstellung und ihre Verwendung als Schädlingsbekämpfungsmittel.

Es ist bereits bekannt, daß bestimmte 1-Arylpyrazole wie beispielsweise das 5-(N-Methylamino)-4-trifluormethylthio-1-(2,6-dichlor-4-trifluormethyl-phenyl)-pyrazol oder das 5-(N-Methylamino)-4-dichlorfluormethylthio-1-(2,6-dichlor-4-trifluormethylphenyl)-pyrazol gute insektizide Eigenschaften besitzen (vgl. EP 201 852).

Die Wirksamkeit dieser vorbekannten Verbindungen ist jedoch insbesondere bei niedrigen Aufwandmengen und Konzentrationen nicht bei allen Schadinsekten sowie nicht in allen Anwendungsbereichen völlig zufriedenstellend.

Es wurden neue substituierte 1-Aryl-5-(het)arylmethylamino-pyrazole der allgemeinen Formel (I),

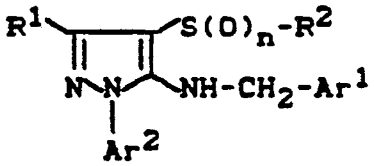

(I)

in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$Ar^1$ für substituiertes Aryl oder für gegebenenfalls substituiertes Heteroaryl steht,
$Ar^2$ für jeweils gegebenenfalls substituiertes Phenyl oder Pyridyl steht und
n für eine Zahl 0, 1 oder 2 steht,
gefunden.

Weiterhin wurde gefunden, daß man die neuen substituierten 1-Aryl-5-(het)arylmethylamino-pyrazole der allgemeinen Formel (I),

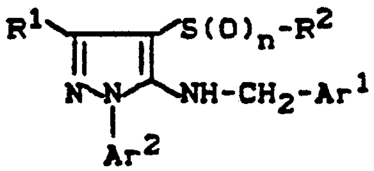

(I)

in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$Ar^1$ für substituiertes Aryl oder für gegebenenfalls substituiertes Heteroaryl steht,
$Ar^2$ für jeweils gegebenenfalls substituiertes Phenyl oder Pyridyl steht und
n für eine Zahl 0, 1 oder 2 steht,
erhält, wenn man 5-Aralkylidenimino-1-arylpyrazole der Formel (II),

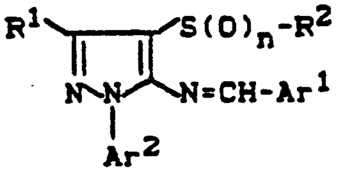

(II)

in welcher
$R^1$, $R^2$, $Ar^1$, $Ar^2$ und n die oben angegebene Bedeutung haben,
mit einem Reduktionsmittel, gegebenenfalls in Gegenwart eines Verdünnungsmittels umsetzt.

Schließlich wurde gefunden, daß die neuen 1-Aryl-pyrazole der allgemeinen Formel (I) eine gute Wirkung gegen Schädlinge besitzen.

Überraschenderweise zeigen die erfindungsgemäßen substituierten 1-Aryl-5-(het)arylmethylamino-pyrazole der allgemeinen Formel (I) eine erheblich bessere insektizide Wirksamkeit als die aus dem Stand der Technik bekannten 1-Arylpyrazole, wie beispielsweise das 5-(N-Methylamino-4-trifluormethylthio-1-(2,6-dichlor-4-trifluormethyl-phenyl)-pyrazol oder das 5-(N-Methylamino-4-dichlorfluormethylthio-1-(2,6-dichlor-4-trifluormethyl-phenyl)-pyrazol, welche chemisch und wirkungsmäßig naheliegende Verbindungen sind.

Die erfindungsgemäßen substituierten 1-Aryl-5-(het)aryl-methylamino-pyrazole sind durch die Formel (I) allgemein definiert. Bevorzugt sind Verbindungen der Formel (I), bei welchen

$R^1$ für Wasserstoff oder für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen steht,

$R^2$ für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil jeweils infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$Ar^1$ für einfach oder mehrfach, gleich oder verschieden substituiertes Phenyl oder für gegebenenfalls einfach oder mehrfach, gleich oder verschieden substituiertes Heteroaryl mit 1 bis 9 Kohlenstoffatomen und 1 bis 3 Heteroatomen, insbesondere Stickstoff, Sauerstoff oder Schwefel steht, wobei als Substituenten jeweils die bei $R^2$ genannten Phenylsubstituenten infrage kommen,

$Ar^2$ für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^3$, wobei

$R^3$ für Amino, sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

p für eine Zahl 0, 1 oder 2 steht und

n für eine Zahl 0, 1 oder 2 steht.

Besonders bevorzugt sind Verbindungen der Formel (I), bei welchen

$R^1$ für Wasserstoff, Methyl, Ethyl, n- oder i-Propyl oder Trifluormethyl steht,

$R^2$ für Methyl, Ethyl, n- oder i-Propyl, n-, i-, s-oder t-Butyl, n- oder i-Pentyl, n- oder i-Hexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl oder für jeweils gegebenenfalls einfach bis dreifach gleich oder verschieden substituiertes Phenyl, Benzyl oder Phenylethyl steht, wobei als Phenylsubstituenten jeweils in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$Ar^1$ für ein- bis dreifach, gleich oder verschieden substituiertes Phenyl oder für jeweils gegebenenfalls ein- bis dreifach, gleich oder verschieden substituiertes Pyridyl, Furyl oder Thienyl steht, wobei als Substituenten jeweils die bei $R^2$ genannten Phenylsubstituenten infrage kommen,

$Ar^2$ für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^3$,

wobei
$R^3$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht,
p für eine Zahl 0, 1 oder 2 steht und
n für eine Zahl 0, 1 oder 2 steht.

Ganz besonders bevorzugt sind Verbindungen der Formel (I), bei welchen
$R^1$ für Wasserstoff oder Methyl steht,
$R^2$ für Methyl, Ethyl, Trifluormethyl, Dichlorfluormethyl oder Chlordifluormethyl steht,
$Ar^1$ für ein- bis dreifach, geich oder verschieden substituiertes Phenyl oder Pyridyl steht, wobei als Substituenten infrage kommen: Fluor, Chlor, Brom, Methyl oder Trifluormethyl,
$Ar^2$ für gegebenenfalls ein- bis fünffach, gleich oder verschieden substituiertes Phenyl steht, wobei als Substituenten infrage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl und
n für eine Zahl 0, 1 oder 2 steht.

Im einzelnen seien außer den bei den Herstellungsbeispielen genannten Verbindungen die folgenden substituierten 1-Aryl-5-(het)arylmethylamino-pyrazole der allgemeinen Formel (I) genannt:

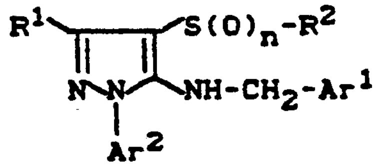

(I)

| $R^1$ | $-S(O)_n-R^2$ | $Ar^1$ | $Ar^2$ |
|---|---|---|---|
| H | $SCCl_2F$ | 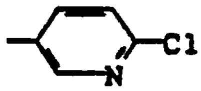 | 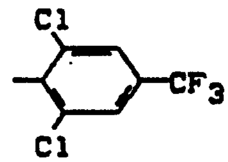 |
| H | $SCClF_2$ | 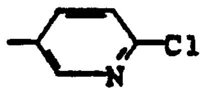 | 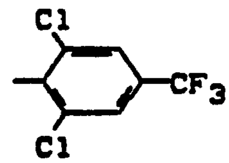 |
| $CH_3$ | $SCF_3$ | 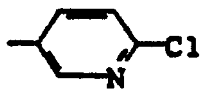 | 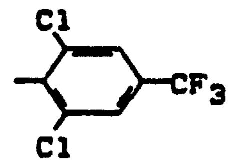 |
| H | $SCF_3$ | 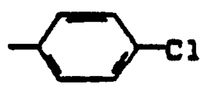 | 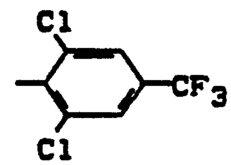 |
| $CH_3$ | $SCF_3$ | 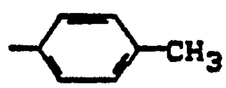 | 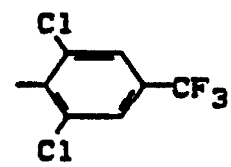 |

Verwendet man beispielsweise 5-[N-[2-Chlorbenzyliden)-imino]-4-trifluormethylthio-1-(2,6-dichlor-4-trifluormethyl-phenyl)-pyrazol als Ausgangsverbindung und Natriumborhydrid als Reduktionsmittel, so läßt sich der Reaktionsablauf des erfindungsgemäßen Verfahrens durch das folgende Formelschema darstellen:

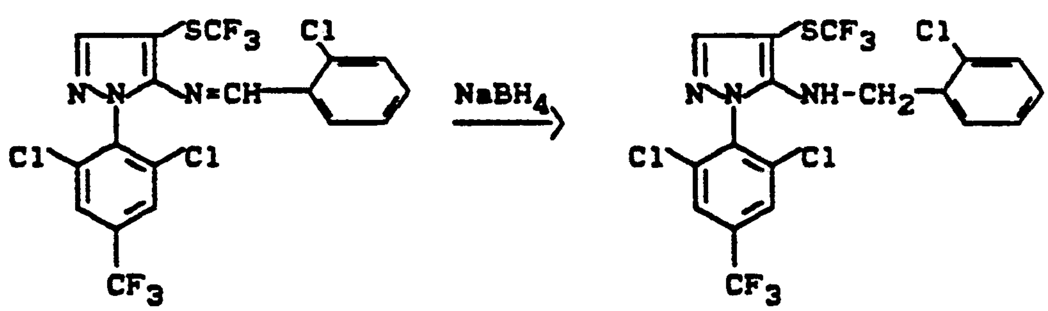


Die zur Durchführung des erfindungsgemäßen Verfahrens als Ausgangsstoffe benötigten 5-Aralkylidenimino-1-arylpyrazole sind durch die Formel (II) allgemein definiert. In dieser Formel (II) stehen $R^1$, $R^2$, $Ar^1$, $Ar^2$ und n vorzugsweise für diejenigen Reste, die bereits im Zusammenhang mit der Beschreibung der erfindungsgemäßen Stoffe der Formel (I) als bevorzugt für diese Substituenten genannt

wurden.

Die 5-Aralkylidenimino-1-aryl-pyrazole der Formel (II) sind noch nicht bekannt. Sie sind jedoch Gegenstand einer eigenen parallelen Patentanmeldung.

Man erhält sie, wenn man 5-Amino-1-aryl-pyrazole der Formel (III),

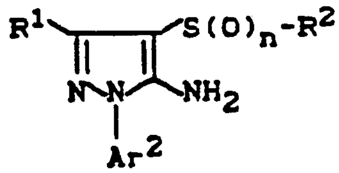

(III)

in welcher

$R^1$, $R^2$, $Ar^2$ und n die oben angegebene Bedeutung haben,

mit Aldehyden der Formel (IV),

$Ar^1$-CHO (IV)

in welcher

$Ar^1$ die oben angegebene Bedeutung hat,

gegebenenfalls in Gegenwart eines Verdünnungsmittels wie beispielsweise Toluol und gegebenenfalls in Gegenwart eines Reaktionshilfsmittels wie beispielsweise Schwefelsäure bei Temperaturen zwischen 0 °C und 120 °C umsetzt.

Die 5-Amino-1-aryl-pyrazole der Formel (III) sind bekannt oder erhältlich in Analogie zu bekannten Verfahren (vgl. EP 201 852).

Als Reduktionsmittel zur Durchführung des erfindungsgemäßen Verfahrens kommen alle für derartige Reduktionsreaktionen üblichen Reduktionsmittel infrage. Vorzugs weise verwendet man komplexe Hydride, wie beispielsweise Natriumborhydrid, Natriumcyanoborhydrid, Lithiumborhydrid oder Lithiumaluminiumhydrid.

Als Verdünnungsmittel zur Durchführung des erfindungsgemäßen Verfahrens kommen in Abhängigkeit vom verwendeteten Reduktionsmittel alle üblichen organischen oder anorganischen Lösungsmittel infrage. Vorzugsweise verwendet man Ether, wie Diethylether, Dioxan oder Tetrahydrofuran oder Alkohole, wie Methanol, Ethanol oder Propanol.

Die Reaktionstemperaturen können bei der Durchführung des erfindungsgemäßen Verfahrens in Abhängigkeit vom verwendeten Reduktionsmittel in einem größeren Bereich variiert werden. Im allgemeinen arbeitet man bei Temperaturen zwischen - 20 °C und + 150 °C, vorzugsweise bei Temperaturen zwischen 0 °C und 120 °C.

Zur Durchführung des erfindungsgemäßen Verfahrens setzt man pro Mol an 5-Aralkylidenimino-1-arylpyrazol der Formel (II) im allgemeinen 0.25 bis 5.0 Mol, vorzugsweise 0.25 bis 2.0 Mol an Reduktionsmittel ein. Die Reaktionsdurchführung, Aufarbeitung und Isolierung der Reaktionsprodukte erfolgt nach allgemein üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis,

Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..

Die erfindungsgemäßen Wirkstoffe zeichnen sich durch eine hohe insektizide Wirksamkeit aus. Sie lassen sich mit gutem Erfolg gegen pflanzenschädigende Insekten, wie beispielsweise gegen die Larven der Meerrettichblattkäfer (Phaedon cochleariae) einsetzen.

Daneben eignen sich die erfindungsgemäßen Wirkstoffe auch zur Bekämpfung von Bodeninsekten und lassen sich beispielsweise zur Bekämpfung der Maden der Zwiebelfliege (Phorbia antiqua) im Boden einsetzen. Außerdem besitzen die erfindungsgemäßen Wirkstoffe eine hohe Wirkung gegen Hygiene- und Vorratsschädlinge.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, als Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste

Trägerstoffe kommen in Frage: z.B. natürliche Gesteinsmehle, wie Kaoline, Tonerden, Talkum, Kreide, Quarz, Attapulgit, Montmorillonit oder Diatomeen-erde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulates aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulf nate sowie Eiweißhydrolysate; als Dispergiermittel komme in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handels üblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die biologische Wirksamkeit der erfindungsgemäßen Verbindungen soll anhand der folgenden Beispiele erläutert werden:

Herstellungsbeispiele

Beispiel 1

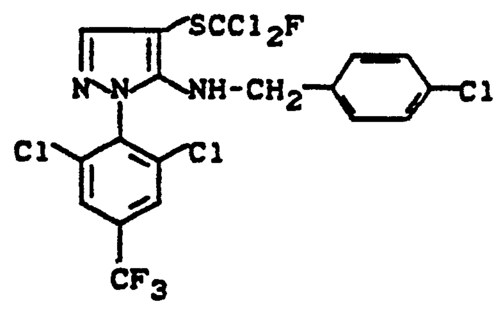

Zu 5,5 g (0,01 Mol) 5-(4-Chlor-benzylidenimino)-1-(2,6-dichlor-4-trifluormethylphenyl)-4-dichlorfluormethylthio-pyrazol in 100 ml Methanol gibt man 0,2 g (0,005 Mol) Natriumborhydrid, rührt 16 Stunden bei Raumtemperatur, engt im Vakuum ein, nimmt den Rückstand in Dichlormethan auf, wäscht zweimal mit Wasser, trocknet über Natriumsulfat, engt im Vakuum ein, kristallisiert den Rückstand durch Verreiben mit Petrolether, saugt ab und trocknet an der Luft.

Man erhält 5,1 g (93 % der Theorie) an 5-(4-Chlorbenzyl-amino)-1-(2,6-dichlor-4-trifluormethyl-phenyl)-4-dichlorfluormethylthio-pyrazol vom Schmelzpunkt 99 °C.

Herstellung der Ausgangsverbindung

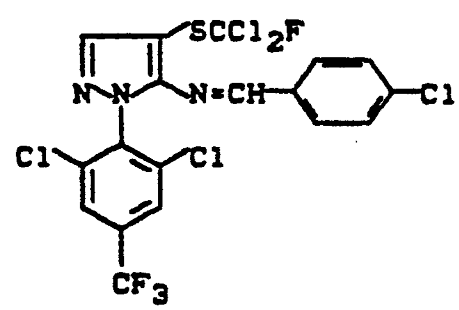

8,6 g (0,02 Mol) 5-Amino-1-(2,6-dichlor-4-trifluormethylphenyl)-4-dichlorfluormethylthio-pyrazol in 200 ml wasserfreiem Toluol werden 4 Stunden über einen Wasserabscheider unter Rückfluß erhitzt. Dann gibt man 4 Tropfen konzentrierte Schwefelsäure zu und tropft innerhalb von 2 Stunden 8,5 g (0,06 Mol) frisch destillierten 4-Chlor-benzaldehyd zu. Nach beendeter Zugabe erhitzt man für weitere 16 Stunden auf Rückflußtemperatur und scheidet dabei freiwerdendes Reaktionswasser über einen Wasserabscheider ab. Zur Aufarbeitung wird die abgekühlte Reaktionsmischung dreimal mit jeweils 100 ml gesättigter Natriumhydrogencarbonatlösung gewaschen, über Natriumsulfat getrocknet und im Vakuum eingeengt. Der Rückstand wird säulenchromatographisch (Kieselgel; Laufmittel: Petrolether/Essigester 9 : 1) gereinigt.

Man erhält 8,5 g (77 % der Theorie) an 5-(4-Chlorbenzylidenimino)-1-(2,6-dichlor-4-trifluormethylphenyl)-4-dichlorfluormethylthio-pyrazol vom Schmelzpunkt 95 °C.

In entsprechender Weise und gemäß den allgemeinen Angaben zur Herstellung erhält man die folgenden 1-Aryl-pyrazole der allgemeinen Formel (I):

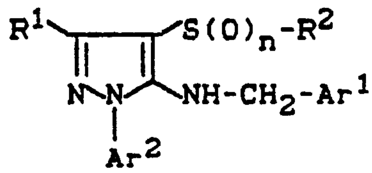

(I)

| Bsp. Nr. | $R^1$ | $-S(O)_n-R^2$ | $Ar^1$ | $Ar^2$ | physikalische Eigenschaften |
|---|---|---|---|---|---|
| 2 | H | $-SCF_3$ | 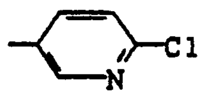 | 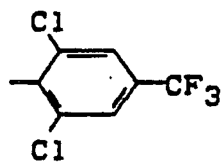 | Fp 103 °C |
| 3 | H | $-SCCl_2F$ | 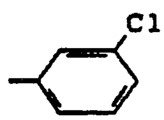 | 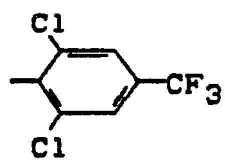 | Fp 93 °C |
| 4 | H | $-SCCl_2F$ | 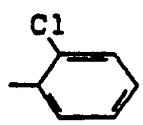 | 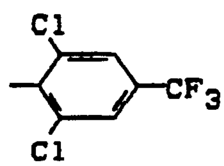 | Fp 138 °C |
| 5 | $CH_3$ | $-SCF_3$ | 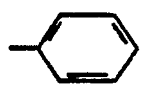 | 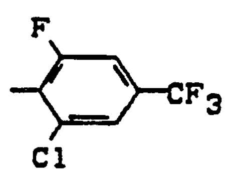 | Fp 86-87° C |

| Bsp. Nr. | $R^1$ | $-S(O)_n-R^2$ | $Ar^1$ | $Ar^2$ | physikalische Eigenschaften |
| --- | --- | --- | --- | --- | --- |
| 6 | $CH_3$ | 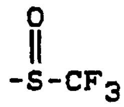 | 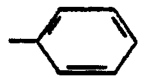 | 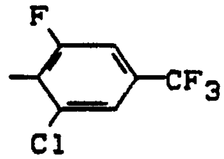 | Fp 96-97° C |
| 7 | $CH_3$ | $-S-CF_3$ | 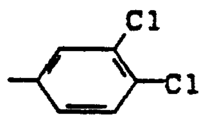 | 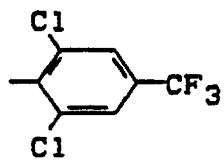 | Fp 94-95° C |
| 8 | $CH_3$ | $-SCCl_2F$ | 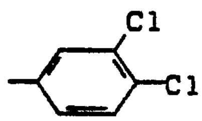 | 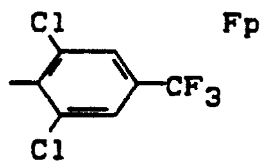 | Fp 105-106° C |
| 9 | $CH_3$ | $-SCCl_2F$ | 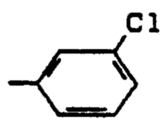 | 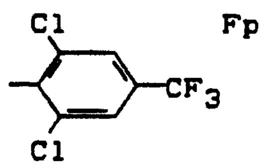 | Fp 109-110° C |

Anwendungsbeispiele

In den folgenden Anwendungsbeispielen wurden die nachstehend aufgeführten Verbindungen als Vergleichsubstanzen eingesetzt:

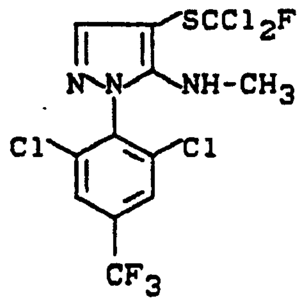

(A)

5-(N-Methylamino)-1-(2,6-dichlor-4-trifluormethyl-phenyl)-4-dichlorfluormethylthio-pyrazol

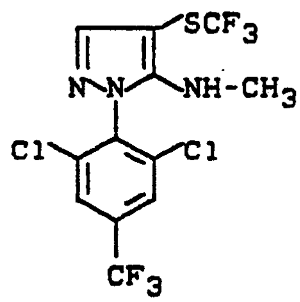

(B)

5-(N-Methylamino)-1-(2,6-dichlor-4-trifluormethyl-phenyl)-4-trifluormethylthio-pyrazol

(beide bekannt aus EP 201 852)

Beispiel A

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die

Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigt z.B. die folgende Verbindung der Herstellungsbeispiele überlegene Wirksamkeit gegenüber dem Stand der Technik: 1.

## Ansprüche

1. Substituierte 1-Aryl-5-(het)arylmethylaminopyrazole der allgemeinen Formel (I)

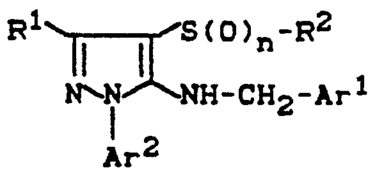

(I)

in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$Ar^1$ für substituiertes Aryl oder für gegebenenfalls substituiertes Heteroaryl steht,
$Ar^2$ für jeweils gegebenenfalls substituiertes Phenyl oder Pyridyl steht und
n für eine Zahl 0, 1 oder 2 steht.

2. Substituierte 1-Aryl-5-(het)arylmethylaminopyrazole der allgemeinen Formel (I) gemäß Anspruch 1, in welcher
$R^1$ für Wasserstoff oder für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen steht,
$R^2$ für geradkettiges oder verzweigtes Alkyl mit 1 bis 8 Kohlenstoffatomen, für geradkettiges oder verzweigtes Halogenalkyl mit 1 bis 8 Kohlenstoffatomen und bis 17 gleichen oder verschiedenen Halogenatomen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil jeweils infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,
$Ar^1$ für einfach oder mehrfach, gleich oder verschieden substituiertes Phenyl oder für gegebenenfalls einfach oder mehrfach, gleich oder verschieden substituiertes Heteroaryl mit 1 bis 9 Kohlenstoffatomen und 1 bis 3 Heteroatomen, insbesondere Stickstoff, Sauerstoff oder Schwefel steht, wobei als Substituenten jeweils die bei $R^2$ genannten Phenylsubstituenten infrage kommen,
$Ar^2$ für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^3$, wobei
$R^3$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,
p für eine Zahl 0, 1 oder 2 steht und
n für eine Zahl 0, 1 oder 2 steht.

3. Substituierte 1-Aryl-5-(het)arylmethylaminopyrazole der allgemeinen Formel (I) gemäß Anspruch 1, in welcher
$R^1$ für Wasserstoff, Methyl, Ethyl, n- oder i-Propyl oder Trifluormethyl steht,
$R^2$ für Methyl, Ethyl, n- oder i-Propyl, n-, i-, s-oder t-Butyl, n- oder i-Pentyl, n- oder i-Hexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Brommethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl oder für jeweils gegebenenfalls einfach bis dreifach gleich oder verschieden substituiertes Phenyl, Benzyl oder Phenylethyl steht, wobei als Phenylsubstituenten jeweils in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$Ar^1$ für ein- bis dreifach, gleich oder verschieden substituiertes Phenyl oder für jeweils gegebenenfalls ein- bis dreifach, gleich oder verschieden substituiertes Pyridyl, Furyl oder Thienyl steht, wobei als Substituenten jeweils die bei $R^2$ genannten Phenylsubstituenten infrage kommen,

$Ar^2$ für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^3$,

wobei

$R^3$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht,

p für eine Zahl 0, 1 oder 2 steht und

n für eine Zahl 0, 1 oder 2 steht.

4. Substituierte 1-Aryl-5-(het)arylmethylaminopyrazole der allgemeinen Formel (I) gemäß Anspruch 1, in welcher

$R^1$ für Wasserstoff oder Methyl steht,

$R^2$ für Methyl, Ethyl, Trifluormethyl, Dichlorfluormethyl oder Chlordifluormethyl steht,

$Ar^1$ ein- bis dreifach, geich oder verschieden substituiertes Phenyl oder Pyridyl steht, wobei als Substituenten infrage kommen: Fluor, Chlor, Brom, Methyl oder Trifluormethyl,

$Ar^2$ für gegebenenfalls ein- bis fünffach, gleich oder verschieden substituiertes Phenyl steht, wobei als Substituenten infrage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl und

n für eine Zahl 0, 1 oder 2 steht.

5. Verfahren zur Herstellung von substituierten 1-Aryl-5-(het)arylmethylamino-pyrazolen der allgemeinen Formel (I)

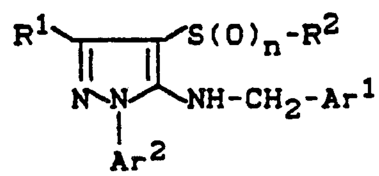

(I)

in welcher

$R^1$ für Wasserstoff, Alkyl oder Halogenalkyl steht,

$R^2$ für Alkyl, Halogenalkyl, für gegebenenfalls substituiertes Aralkyl oder für gegebenenfalls substituiertes Aryl steht,

$Ar^1$ für substituiertes Aryl oder für gegebenenfalls substituiertes Heteroaryl steht,

$Ar^2$ für jeweils gegebenenfalls substituiertes Phenyl oder Pyridyl steht und

n für eine Zahl 0, 1 oder 2 steht,

dadurch gekennzeichnet, daß man 5-Aralkylidenimino-1-arylpyrazole der Formel (II),

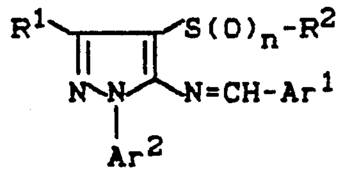

(II)

in welcher
$R^1$, $R^2$, $Ar^1$, $Ar^2$ und n die oben angegebene Bedeutung haben, mit einem Reduktionsmittel, gegebenenfalls in Gegenwart eines Verdünnungmittels umsetzt.

6. Schädlingsbekämpfungsmittel, gekennzeichnet durch einen Gehalt an mindestens einem substituierten 1-Aryl-5-(het)arylmethylamino-pyrazol der Formel (I).

7. Insektizide Mittel, gekennzeichnet durch einen Gehalt an mindestens einem substituierten 1-Aryl-5-(het)arylmethylamino-pyrazol der Formel (I).

8. Verfahren zur Bekämpfung von tierischen Schädlingen, insbesondere von Insekten, dadurch gekennzeichnet, daß man substituierte 1-Aryl-5-(het)arylmethylamino-pyrazole der Formel (I) auf tierische Schädlinge und/oder ihren Lebensraum einwirken läßt.

9. Verwendung von substituierten 1-Aryl-5-(het)arylmethylamino-pyrazolen der Formel (I) zur Bekämpfung von tierischen Schädlingen, insbesondere von Insekten.

10. Verfahren zur Herstellung von Mitteln gegen tierische Schädlinge, dadurch gekennzeichnet, daß man substituierte 1-Aryl-5-(het)arylmethylaminopyrazole der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 88111483.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | DE - A1 - 3 606 476 (BAYER AG)<br>* Ansprüche *<br>-- | 1,5-10 | C 07 D 231/44<br>C 07 D 401/12<br>A 01 N 43/56<br>//C 07 D 401/12<br>C 07 D 231:44<br>C 07 D 213:61) |
| A | WO - A1 - 87/03 781 (MAY & BAYER LIMITED)<br>* Ansprüche 1,5,10,12 *<br>-- | 1,5-10 | |
| A | CHEMICAL ABSTRACTS, Band 99, Nr. 11, 12. September 1983, Columbus, Ohio, USA<br>GIORI, P. et al. "Synthesis and antifungal activity of pyrazole derivatives containing sulfurated functions"<br>Seite 562, Spalte 1, Zusammenfassung-Nr. 88 101m<br>& Farmaco, Ed. Sci. 1983, 38(4), 274-82<br>-- | 1,5-10 | **RECHERCHIERTE SACHGEBIETE (Int. Cl.4)** |
| A | CHEMICAL ABSTRACTS, Band 75, Nr. 5, 2. August 1971, Columbus, Ohio, USA<br>GIORI, Paolo et al. "Synthesis and antifungal properties of pyrazolyl mono- and disulfides. II."<br>Seite 498, Spalte 2, Zusammenfassung-Nr. 35 870j<br>& Farmaco, Ed. Sci. 1971, 26(4), 276-93<br>---- | 1,5-10 | C 07 D 231/00<br>C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 04-11-1988 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82